(19) Europäisches Patentamt
European Patent Office
Office européen des brevets

(11) Numéro de publication: **0 371 878 A1**

(12) **DEMANDE DE BREVET EUROPEEN**

(21) Numéro de dépôt: **89403307.5**

(22) Date de dépôt: **29.11.89**

(51) Int. Cl.5: **C08B 37/08, A23K 1/16, A23K 1/18, A61K 7/00, C02F 1/28, D01F 9/00, D06M 15/00**

(30) Priorité: **30.11.88 FR 8815676**
**30.11.88 FR 8815675**

(43) Date de publication de la demande:
**06.06.90 Bulletin 90/23**

(84) Etats contractants désignés:
**AT BE CH DE ES FR GB GR IT LI LU NL SE**

(71) Demandeur: **RHONE-POULENC SANTE**
**20, avenue Raymond Aron**
**F-92160 Antony(FR)**

(72) Inventeur: **Franzoni, Christine**
**14 Quai de la Pêcherie**
**F-69001 Lyon(FR)**
Inventeur: **Gagnieu, Christian**
**1 rue Victor Despeignes**
**F-69008 Lyon(FR)**
Inventeur: **Porte, Hugues**
**6 Chemin de Crépieux**
**F-69300 Caluire(FR)**

(74) Mandataire: **Le Pennec, Magali et al**
**RHONE-POULENC SANTE, Service Brevets,**
**20 Avenue Raymond Aron**
**F-92165 Antony Cédex(FR)**

(54) **Compositions pour l'enrobage d'additifs alimentaires destinés aux ruminants.**

(57) Nouveaux dérivés organo-solubles du chitosan, leur procédé de préparation ainsi que les nouvelles compositions pour l'enrobage d'additifs alimentaires destinés aux ruminants qui sont stables à un pH supérieur à 5 et qui libèrent la substance biologiquement active à un pH inférieur à 3,5 contenant comme substance sensible aux variations du pH un dérivé organosoluble du chitosan constitué par l'enchaînement statistique de motifs représentés par les formules :

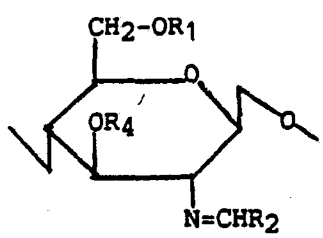

(Ia)

et

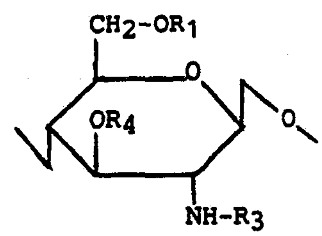

(Ib)

dans lesquelles :
$R_1$ représente un radical alkylcarbonyle (2 à 4 atomes de carbone),
$R_2$ représente un radical alkyle (2 à 21 atomes de carbone) ou phényle éventuellement substitué,
$R_3$ et $R_4$ représentent un atome d'hydrogène ou des radicaux alkylcarbonyle (2 à 4 atomes de carbone).

EP 0 371 878 A1

## COMPOSITIONS POUR L'ENROBAGE D'ADDITIFS ALIMENTAIRES DESTINES AUX RUMINANTS

La présente invention concerne de nouveaux dérivés organosolubles du chitosan, leur procédé de préparation ainsi que les compositions pour l'enrobage d'additifs alimentaires ou de substances biologiquement actives destinés aux animaux manogastriques ou polygastriques contenant comme substances sensibles aux variations du pH ces nouveaux dérivés organosolubles du chitosan.

Le chitosan est un polysaccharide basique, dont la masse moléculaire moyenne est généralement supérieure à 500.000, qui est constitué de résidus amino-2 désoxy-2 β-D-glucopyranose liés en 1,4. Le chitosan peut être obtenu par désacétylation de la chitine qui peut elle-même être extraite par exemple de la carapace des crustacés où elle se trouve en quantité importante.

Du fait de sa structure chimique, le chitosan présente la propriété d'être résistant à l'hydrolyse au niveau des liaisons osidiques en milieu basique, d'être insoluble à un pH supérieur à 6,5 et d'être soluble et hydrolysable dans des conditions acides. Compte tenu de ses propriétés filmogènes, le chitosan pourrait être un agent de choix pour protéger diverses substances dont la libération doit être régulée en fonction du pH. Cependant, le chitosan présente l'inconvénient d'être insoluble dans les solvants organiques usuels ce qui rend difficile la réalisation de films ou de pellicules d'enrobage.

Il a maintenant été trouvé, et c'est ce qui fait l'objet de la présente invention, de nouveaux dérivés du chitosan qui sont organosolubles et qui conservent la propriété de former des films et d'être sensibles aux variations du pH.

Les nouveaux dérivés du chitosan selon l'invention sont constitués par l'enchaînement statistique de motifs qui peuvent être représentés par les formules suivantes :

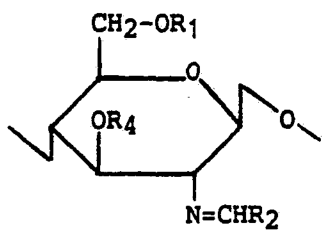

(Ia)

et

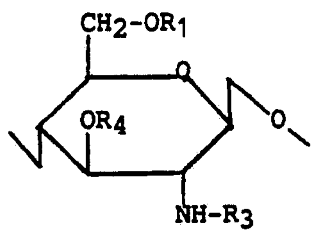

(Ib)

(I)

dans lesquelles :

- $R_1$ représente un radical alkylcarbonyle contenant 2 à 4 atomes de carbone
- $R_2$ représente un radical alkyle contenant 2 à 21 atomes de carbone ou un radical phényle éventuellement substitué par un ou plusieurs radicaux hydroxy ou alcoxy,
- $R_3$ et $R_4$ représentent des groupes identiques ou différents choisis parmi les radicaux alkylcarbonyle contenant 2 à 4 atomes de carbone et l'hydrogène.

Le chitosan tel que défini par les formules (I) a de préférence une masse moléculaire moyenne comprise entre 10.000 et 80.000 et encore plus préférentiellement comprise entre 10.000 et 20.000.

Il a également de préférence une composition correspondant a :

60 à 100 % de motifs répondant à la formule (Ia)

0 à 40 % de motifs répondant à la formule (Ib)

Les dérivés du chitosan de l'invention qui sont préférés sont ceux pour lesquels dans les composés de formule (I) au moins l'une, et de préférence plusieurs, des conditions suivantes sont remplies :

au moins 50 % des motifs $R_1$ représentent un radical acétyle

$R_2$ représente un radical alkyle contenant 5 à 12 atomes de carbone

au moins 50 % des motifs $R_3$ représentent un radical acétyle

au moins 50 % des motifs $R_4$ représentent un radical acétyle

Le procédé de préparation des nouveaux dérivés du chitosan consiste à utiliser un chitosan ayant un degré de désacétylation supérieur à 80 %, puis :

- dans une première étape à hydrolyser ce chitosan,
- dans une deuxième étape à condenser un aldéhyde aliphatique ou aromatique avec le chitosan issu de la première étape,
- dans une troisième étape à estérifier le chitosan issu de la deuxième étape.

La désacétylation est réalisée à partir de chitine issue de carapaces d'animaux par mise en contact avec de l'hydroxyde de sodium en solution aqueuse pendant environ 1 heure à environ 135° C.

Postérieurement à la désacétylation et préalablement à l'étape d'hydrolyse, il est avantageux de purifier le chitosan par traitement à l'acide acétique, précipitation avec une base forte, de préférence la soude, lavage à l'eau et à l'éthanol puis chauffage dans l'éthanol à environ 80 °C. Cette technique permet une purification du chitosan technique.

Selon un mode préférentiel de mise en oeuvre de la première étape, on hydrolyse le chitosan avec un acide fort choisi parmi :

- l'acide chlorhydrique
- l'acide sulfurique
- l'acide nitrique
- les acides minéraux supportés (type TONSIL r).

Selon un procédé de mise en oeuvre de cette première étape, on introduit dans une solution d'acide fort, d'acidité 0,5 N à 1 N, environ 25 à 50 g/litre de chitosan.

Selon un mode préférentiel de mise en oeuvre l'hydrolyse est réalisée à une température comprise entre 80 et 120 °C et de préférence à environ 100 °C pendant 5 à 30 heures.

Le chitosan hydrolysé est ensuite précipité par alcalinisation à un pH voisin de 10. Il est alors séparé par filtration.

Les polysaccharides obtenus après hydrolyse présentent une masse moléculaire moyenne notamment comprise entre 10.000 et 80.000 et tout particulièrement entre 10.000 et 20.000.

Selon un mode préférentiel de mise en oeuvre de la deuxième étape on condense sur le chitosan hydrolysé obtenu à la première étape un aldéhyde de formule (II)

$R_2$-CHO (II)

dans laqielle $R_2$ a la même signification que précédemment.

La condensation de l'aldéhyde et du chitosan hydrolysé est effectuée de préférence à un pH compris entre 5,5 et 6 notamment d'environ 5,5.

Le solvant de condensation est de préférence un milieu hydroalcoolique contenant un alcool choisi parmi le méthanol, l'éthanol ou l'isopropanol. Le rapport pondéral eau/alcool est de préférence compris entre 0,45 et 0,65.

On préfère mettre en oeuvre parmi les aldéhydes de formule (II) les aldéhydes aliphatiques ayant 3 à 14 atomes de carbone tels que :

- le propanal,
- le butanal,
- le pentanal,
- l'hexanal,
- l'heptanal,
- le décanal,
- le dodecanal,
- le tetradecanal.

Pour une meilleure mise en oeuvre de l'invention, on préfère que le rapport molaire de l'aldéhyde de formule (II) au motif monomère osidique moyen de chitosan soit supérieur à 15 et de préférence d'environ 20.

La condensation est effectuée selon un mode de mise en oeuvre préférentiel entre 10 et 50 °C et encore plus préférentiellement entre 10 et 30 °C.

Le produit de condensation de l'aldéhyde sur le chitosan issu de la deuxième étape du procédé de l'invention est extrait notamment au moyen de solvants organiques choisis parmi :

- les cétones telles que l'acétone,
- les alcools tels que l'éthanol.

La troisième étape du procédé de l'invention consiste à estérifier le chitosan modifié issu de la deuxième étape.

La réaction d'estérification consiste à mettre en présence un acide ou un dérivé d'acide de formule (III)

$(R\,CO)_nA$ (III)

dans laquelle :

n est égal à 1 ou 2

A est égal à un groupe hydroxyle ou un halogène quand $n = 1$

A est égal à l'oxygène quand n est égal à 2

R représente un groupe alkyle ayant un à trois atomes de carbone avec le chitosan modifié issu de la deuxième étape.

L'acylation est réalisée de préférence avec un acide ou un dérivé d'acide de formule (III) dans lequel R est égal à un groupe alkyle contenant 1 à 3 atomes de carbone et tout particulièrement dans lequel R est

égal au groupe - $CH_3$.

On préfère utiliser parmi les dérivés d'acide de formule (III) les anhydrides d'acides et tout préférentiellement l'anhydride acétique.

La réaction d'acylation est réalisée de préférence dans un solvant organique tel que les pyridines.

Lorsque la réaction est réalisée entre un halogénure d'acide et le chitosan, on préffère ajouter une base de façon à neutraliser l'hydracide formé ou on utilise un solvant organique basique comme par exemple les pyridines.

L'acylation est réalisée notamment à une température comprise entre 10 et 50 ° C, et de préférence à une température comprise entre 10 et 30 ° C.

Le chitosan modifié issu de la troisième étape est récupéré par exemple par évaporation du solvant de réaction, puis après lavage en milieu basique, le produit est repris à l'acétone de façon à éliminer par centrifugation la partie insoluble.

Le chitosan obtenu présente une masse moléculaire moyenne notamment comprise entre 10.000 et 110.000 et tout particulièrement entre 10.000 et 25.000.

Le chitosan modifié est utilisable :

- comme composant dans les préparations cosmétiques,
- comme agent de séquestration des métaux lourds avec formation d'un complexe insoluble,
- pour la préparation de films, de fils, de fibres, de revêtements,
- pour l'enrobage d'additifs alimentaires ou de substances biologiquement actives destinés à l'alimentation des ruminants:

Les dérivés du chitosan selon la présente invention présentent la propriété d'être solubles dans les solvants organiques usuels tels que l'acétone, l'éthanol ou le chlorure de méthylène. D'un intérêt tout particulier sont les dérivés du chitosan constitués de l'arrangement statistique des motifs de formules générales (Ia) et (Ib) dans lesquelles $R_2$ représente un radical alkyle contenant 2 à 13 atomes de carbone et plus spécialement 6 à 9 atomes de carbone, le taux de fixation de l'aldéhyde étant compris entre 55 et 65 % par rapport aux monomères osidiques du chitosan, $R_1$ représente un radical acétyle, $R_3$ représente un atome d'hydrogène ou un radical acétyle et une partie des radicaux $R_4$ représente un atome d'hydrogène et l'autre un radical acétyle.

Par exemple, l'organosolubilité qui correspond à la fraction des produits organosolubles dans un produit brut issu de l'enchaînement réactionnel, peut être déterminée en mettant 10 g de produit brut d'acétylation broyé dans 100 g de solvant et en mesurant la quantité de produit qui se solubilise.

Les dérivés du chitosan selon l'invention sont particulièrement utiles pour préparer des compositions pour l'enrobage des additi fs al imentaires ou des substances biologiquement actives destinés principalement aux ruminants qui sont stables dans un milieu dont le pH est égal ou supérieur à 5,5 et qui permettent la libération de l'additif alimentaire ou de la substance biologiquement active dans un milieu dont le pH est inférieur ou égal à 3,5.

Lorsque l'on administre à des ruminants certaines substances biologiquement actives (médicaments, vitamines, aminoacides), il se produit, lors du passage dans le rumen, une destruction enzymatique de ces substances favorisée par le temps de séjour (quelques heures à plusieurs jours) et par le pH (compris entre 5 et 6). Il en résulte que la substance active qui est dégradée perd la majeure partie de son efficacité lorsqu'elle arrive dans la caillette et l'intestin du ruminant.

Il importe donc de pouvoir protéger ces substances biologiquement actives par des enrobages qui soient stables dans le rumen des ruminants, c'est-à-dire qui soient stables à la dégradation par les microorganismes et qui permettent la libération des substances biologiquement actives dans une partie de l'appareil digestif, plus particulièrement la caillette, dont le pH est inférieur ou égal à 3,5. Alors que la durée de protection dans le rumen doit être relativement longue (quelques heures à plusieurs jours), la libération de la substance active dans la caillette doit s'effectuer dans un temps relativement court (quelques minutes à quelques heures).

Les nouveaux dérivés du chitosan selon la présente invention peuvent être utilisés comme substances pH sensibles dans les compositions d'enrobage d'additifs alimentaires ou de substances biologiquement actives destinés aux ruminants. En particulier, les nouveaux dérivés organosolubles du chitosan peuvent remplacer avantageusement, totalement ou partiellement, les substances pH sensibles synthétiques telles que les copolymères basiques comme les copolymères du styrène avec les vinylpyridines qui sont habituellement utilisées dans ce type de composition.

Les dérivés du chitosan selon l'invention, qui sont issus de produits naturels acceptables dans l'alimentation, sont hydrolysés dans l'organisme en substances non toxiques pour les animaux, ce qui représente un avantage pratique considérable.

Plus spécialement, les nouveaux dérivés du chitosan peuvent être utilisés comme substances pH

sensibles dans les compositions d'enrobage qui font l'objet des brevets français FR 2 514 261, FR 2 582 909, FR 2 575 039, FR 2 575 040, FR 2 603 458 ou FR 2 606 597, et ils peuvent être mis en oeuvre de la même manière.

Les exemples suivants, donnés à titre non limitatif, illustrent la présente invention.

EXEMPLE 1

1 - Purification du chitosan

On dissout 40 g de chitosan technique (de la Société S1GMA) dans 3 litres d'une solution aqueuse d'acide acétique à 4 % (p/v). La solution est filtrée sur un tamis dont la maille est de 125 microns. Le chitosan est précipité par addition d'une solution aqueuse de soude à 25 jusqu'à pH 9-11 puis séparé par filtration puis lavé sur tamis par de l'eau distillée et enfin par de l'éthanol absolu. Le précipité fibreux est pressé pour extraire le maximum de solvant puis il est dispersé dans 1 litre d'éthanol absolu. Le mélange est chauffé à 80° C pendant 2 heures. Après filtration, le chitosan purifié est séché sous pression réduite à 60° C. On obtient ainsi 35 g de chitosan purifié dont les caractéristiques sont les suivantes :
- spectre infra-rouge (à partir de comprimés en mélange avec KBr) : principales bandes d'absorption caractéristiques à 3400, 2900 et 1650 $cm^{-1}$
- masse moléculaire moyenne : supérieure à 500.000.

La masse moléculaire moyenne est déterminée par chromatographie liquide à haute performance en utilisant 5 colonnes de 50 cm de long remplies de glycophase G/CPG (N.D. Pierce) de porosité 3125 (2 colonnes), 1902, 1038 et 547 Å. La phase mobile est un tampon acétate de sodium 0,2M et acide acétique 0,33M à pH = 4,2. Le débit est de 1 cm3/minute et la détection se fait par réfractométrie. L'étalonnage est fait par des étalons de dextran (de la Société S1GMA) de masse moléculaire de 506.000 à 110.000.
- pourcentage de désacétylation : 80 % des cycles osidiques du chitosan purifié contiennent une fonction amine primaire libre.

Le pourcentage de désacétylation est déterminé de la manière suivante : un échantillon de chitosan réduit en poudre (particules de 20 microns environ) est mis en suspension dans un mélange eau-diméthylsulfoxyde (9-1 en volumes) dont le pH est amené à 11 par addition de soude 0,1N. Le dosage potentiométrique est effectué par de l'acide chlorhydrique 0,1N.
- analyse élémentaire :
C % = 40,90 H % = 6,76 N % = 7,52 O % = 44,82

2 - Hydrolyse acide du chitosan purifié

On dissout 25 g de chitosan purifié dans 650 cm3 d'acide chlorhydrique 0,5N. La solution est chauffée dans un bain d'huile à 98° C pendant 18 heures. Après refroidissement, le chitosan est précipité lentement par addition de soude 5N jusqu'à ce que le pH du milieu réactionnel atteigne 10. Les sels formés sont éliminés par dialyse. Le chitosan purifié est séparé par filtration ou centrifugation, lavé par de l'éthanol absolu puis séché sous pression réduite à 60° C. On obtient ainsi 21 g de chitosan hydrolysé dont les caractéristiques sont les suivantes :
- masse moléculaire moyenne : voisine de 13.000 (détermination dans les conditions décrites précédemment en utilisant 4 colonnes de cm de long remplies de glycophase G/CPG (H.D. Pierce) de porosité 1902, 1038, 547 et 242 Å et des étalons de dextran (de la Société S1GMA) de masse moléculaire de 110.000 à 9.000.
- analyse élémentaire :
C % = 40,13 H % = 6,76 N % = 7,72 O % = 45,39

3 - Condensation du décanal sur le chitosan hydrolysé

On dissout 1 g de chitosan hydrolysé dans 20 cm3 d'une solution aqueuse d'acide acétique à 10 %. Le pH est ajusté à 4,5 par addition d'une solution de soude 5N puis on ajoute 40 cm3 de méthanol et 23 cm3 de décanal. Le mélange réactionnel visqueux est agité pendant 18 heures à une température voisine de 20° C. Le produit de condensation est extrait à l'acétone dans un appareil de Soxhlet pendant 5 heures.

Après séchage sous pression réduite à 35 °C, on obtient 1,48 g du produit de condensation décanal-chitosan dont les caractéristiques sont les suivantes :
- spectre infra-rouge (détermination à partir de comprimés en mélange avec KBr) : principales bandes d'absorption caractéristiques à 3450, 2930 et 2860 $cm^{-1}$
- pourcentage de fixation du décanal : 60-65 %.

La détermination est effectuée après réduction de la liaison imine par le borohydrure de sodium en milieu éthanol-eau (1-1 en volumes). Le pourcentage de groupements amines secondaires est déterminé par dosage potentiométrique du dérivé réduit dissous en milieu acide acétique-éthanol (2-1 en volumes) en titrant par l'acide perchlorique 0,1N.

4 - Acétylation du produit de condensation décanal-chitosan

On disperse 1 g du produit de condensation décanal-chitosan dans 25 cm3 de pyridine anhydre. On ajoute 5 cm3 d'anhydride acétique. Le mélange réactionnel est agité pendant 24 heures à une température voisine de 20 °C. Après évaporation de la pyridine, le résidu est repris deux fois par du toluène puis mis en suspension dans du pentane. La suspension est filtrée puis lavée 3 fois avec une solution de soude 0,1N puis rincée à l'eau distillée jusqu'à neutralité. Après transfert dans un ballon, le produit est séché par entraînement de l'eau à l'acétone.

Le produit obtenu est purifié par centrifugation à 16.000 tours/minute après dissolution dans 50 cm3 d'acétone. Après évaporation du surnageant, le résidu obtenu est lavé sur verre fritté n° 3 avec de l'éther éthylique.

On obtient ainsi 1,075 g de produit de formule générale (I) constitué de l'arrangement statistique des motifs de formule :

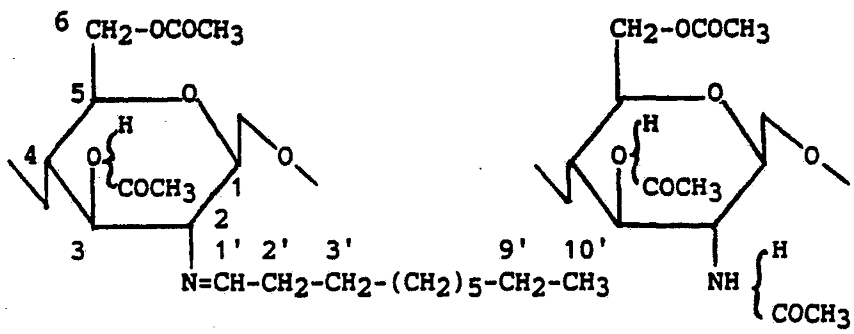

dont les caractéristiques sont les suivantes :
- pourcentage de fixation de l'aldéhyde : 60-65 %
- pourcentage d'acétylamino : 20 à 35-40 %
- spectre infra-rouge (détermination à partir de comprimés en mélange avec KBr) : principales bandes d'absorption caractéristiques à 3400, 2920-2830, 1750-1230, 1680, 1640 et 1540 $cm^{-1}$
- spectre de résonance magnétique du 13C (90 MHZ - chloroforme deutéré - déplacement en ppm) : 170 (CO de l'acétyle) - 169 (CH=N) -102 (C-1) - 72,73 et 75,5 (C-2, C-3, C-4 et C-5) - 62,5 (C-6) - 31,8 ($CH_2$ en 2′) - 29,4 [$(CH_2)_5$ de la chaîne aldéhydique] - 25 ($CH_3$ de $CH_3CO$) - 22,6 ($CH_2$ en 9′) - 20,8 ($CH_2$ en 3′) - 14,0 ($CH_3$ en 10′)
- analyse élémentaire :
C % = 61,00 H % = 8,17 N % = 3,28 O % = 27,55
- masse moléculaire Mw = 15300 avec indice de polydispersité de 7,14.

La masse moléculaire Mw est déterminée par filtration sur gel en utilisant 6 colonnes en série : une précolonne de 5 cm de long (PL gel de 100 Å), une colonne de 50 cm (Shodex A 801), une colonne de 30 cm (PL gel $10^6$ Å), une colonne de 60 cm (PL gel 100 Å) et deux colonnes mixtes de 60 cm (PL gel). La chromatographie est effectuée dans le dichlorométhane avec un débit de 1,5 cm3/minute. La détection se fait par réfractométrie. On utilise des étalons de polystyrène de Mw 100 à $4.10^6$.

Le produit est soluble dans l'acétone (30 à 40 en poids) l'éthanol (15 en poids) et le dichlorométane (10 % en poids).

5 - Utilisation pour former un film

On dissout 40 g de chitosan obtenu précédemment dans 100 ml d'un mélange éthanol-dichloro 1,2 éthane (50/50 en poids). On ajoute 5 % en poids à la solution précédente de triacétate de glycérol (plastifiant alimentaire).

On coule la solution obtenue sur une plaque de polyéthylène. On laisse évaporer le solvant à l'air libre. On obtient un film ayant une épaisseur variant de 50 à 120 microns. On découpe 8 éprouvettes Hz. On leur fait subir des tractions de 1 mm/mn dans un appareil INSTROM. On mesure les caractéristiques suivantes :
- module d'élasticité : 230 + 50 MPa
- caractéristiques à la rupture force : 1,8 + 0,8 N
contrainte : 6,3 + 1,8 MPa
allongement: 4,7 + 2 %

A l'examen au microscope électronique n'apparaît ni fissure ni porosité autant sur le film sans plastifiant qu'avec plastifiant.

EXEMPLES 2 A 6

En opérant comme dans l'exemple 1, à partir de 1 g de chitosan hydrolysé, et en utilisant différents aldéhydes aliphatiques, les résultats qui sont obtenus sont rassemblés dans le tableau 1.

TABLEAU 1

| Exemples | Aldéhyde | Pourcentage de fixation de l'aldéhyde | Poids du produit de condensation aldéhyde-chitosan | Poids du produit de condensation aldéhyde-chitosan acétylé |
|---|---|---|---|---|
| 2 | propanal (9 cm3) | 69 | 1,20 g | 0,160 g |
| 3 | pentanal (13 cm3) | 63 | 1,36 g | 0,580 g |
| 4 | heptanal (17 cm3) | 61 | 1,49 g | 0,965 g |
| 5 | dodécanal (27 cm3) | 58 | 1,70 g | 0,900 g |
| 6 | tétradécanal (25 g) | 59 | 1,82 g | 0,600 g |

EXEMPLE 7

On dissout 1 g de chitosan hydrolysé dans 20 cm3 d'une solution aqueuse d'acide acétique à 10 %. Le pH du milieu est ajusté à 4,5 par addition d'une solution de soude 5N. On ajoute 10 cm3 de méthanol puis une solution de 18 g d'hydroxy-4 méthoxy-3 benzaldéhyde dans 30 cm3 de méthanol. Le mélange réactionnel vert foncé est agité pendant 18 heures à une température voisine de 20 °C. Le précipité formé est extrait à l'acétone dans un appareil de Soxhlet pendant 5 heures. Après séchage à 35 °C sous pression réduite, on obtient 1,65 g du produit de condensation de l'hydroxy-4 méthoxy-3 benzaldéhyde avec le chitosan hydrolysé qui est acétylé dans les conditions décrites dans l'exemple 1.

Après purification, on obtient 0,990 g de produit de formule générale (1) constitué de l'enchaînement statistique des motifs de formule :

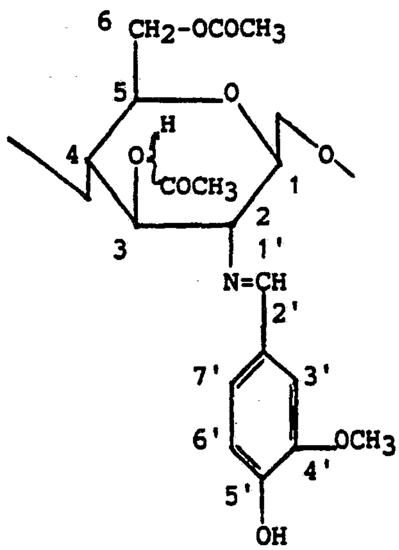

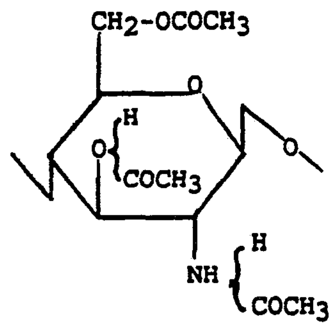

dont les caractéristiques sont les suivantes :

- pourcentage de fixation de l'aldéhyde : 60-65 %
- pourcentage d'acétylamino : 20 à 35-40 %
- spectre infra-rouge (détermination à partir de comprimés en mélange avec KBr) : principales bandes d'absorption caractéristiques à 3400, 2945-2873, 1745-1227, 1691, 1647 et 1602-1507 $cm^{-1}$
- spectre de résonance magnétique nucléaire du 13C (90 MHz - choroforme deutéré - déplacement en ppm) : 170,1 (CO de l'acétyle) - 152 (C-5') - 151,8 (C-4') - 135,3 (C-2') - 124,6 (C-7') - 123,4 (C-6') -111,0 (C-3') - 102,2 (C-1) - 75,9 à 72,4 (C-2, C-3, C-4 et C-5) -62,5 (C-6) - 56,1 ($CH_3$-O).

EXEMPLE 8

Selon la technique du lit fluidisé ("spray-coating") avec une cuve équipée d'un système WURSTER, on enrobe 350 g de méthionine préalablement granulée sous forme de particules sphériques titrant 98 % dont le diamètre moyen est compris entre 0,63 et 0,80 mm par une solution dont la composition est la suivante :

| | |
|---|---|
| - acide stéarique (P.F. = 68-69 °C ; indice d'acide 194-198).. | 88 g |
| - chitosan modifié selon l'exemple 1 ... | 22 g |
| - dichlorométhane ... | 500 cm3 |
| - éthanol ... | 500 cm3 |

La solution, maintenue à 28° C, est pulvérisée en 60 minutes.
On obtient ainsi 449 g de granulés titrant 75 % en méthionine

EXEMPLE 9

Selon la technique du lit fluidisé avec une cuve équipée d'un système WURSTER; on enrobe 350 g de chlorhydrate de lysine préalablement granulé sous forme de particules sphériques dont le diamètre moyen est voisin de 0,8 mm par une solution dont la composition est identique à celle décrite dans l'exemple 8.

La solution, maintenue à 29° C, est pulvérisée en 1 heure 34 minutes.

On obtient ainsi 448 g de granulés titrant 70 % en chlorhydrate de lysine.

Pour mettre en évidence la sensibilité des compositions d'enrobage aux variations du pH, des tests sont utilisés qui permettent de mesurer la libération de la matière active en fonction du temps à différentes valeurs du pH et, notamment, à pH = 6 et à pH = 2.

Par exemple, le relargage de la substance active présente dans les granulés enrobés est examiné en agitant, dans des conditions déterminées, une quantité connue de granulés dans le milieu tamponné maintenu à pH constant à une température de 40° C. On compare les cinétiques de libération d'un échantillon à différentes valeurs du pH et plus particulièrement à pH = 6 et à pH = 2.

Avec les granulés qui font l'objet des exemples 8 et 9, les résultats obtenus sont rassemblés dans le tableau 2 .

TABLEAU 2

| Exemples | Titre en substance active | % de substance active libérée | | | | | | | |
|---|---|---|---|---|---|---|---|---|---|
| | | à pH = 6 après | | | | à pH = 2 après | | | |
| | | 1 h | 6 h | 24 h | 15 min | 1 h | 2 h | 3 h | 5 h |
| 8 | 75 % (méthionine) | 0 | 1,2 | 3,0 | 0,7 | 1,1 | 7,3 | 44 | 100 |
| 9 | 70 % (chlorhydrate de lysine) | 2,7 | 10,0 | 20,0 | 1,2 | 3,4 | 37 | 100 | |

L'efficacité in vivo des compositions d'enrobage selon l'invention peut être mise en évidence dans le test suivant :

Des échantillons de granulés enrobés (environ 0,5 g) sont introduits dans des sachets en nylon ayant une maille de 300 x 300 microns. Les sachets sont placés dans le rumen de brebis fistulées pendant 6 heures, 15 heures et 24 heures. Les sachets sont récupérés et lavés. La quantité de substance active présente dans les sachets est déterminée par une méthode appropriée.

Les résultats obtenus sont rassemblés dans le tableau 3.

TABLEAU 3

| Temps de séjour dans le rumen | % de méthionine résiduelle Produit de l'exemple 8 | % de lysine résiduelle Produit de l'exemple 9 |
|---|---|---|
| 6 | 99 ± 1 | - |
| 15 | 98,7 ± 1 | 71 ± 7 |
| 24 | 96,8 ± 0,6 | 67,5 ± 2,5 |

**Revendications**

1 - Nouveaux dérivés organosolubles du chitosan caractérisés en ce qu'ils sont constitués par

l'enchaînement statistique de motifs de formule :

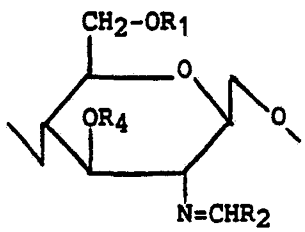 et 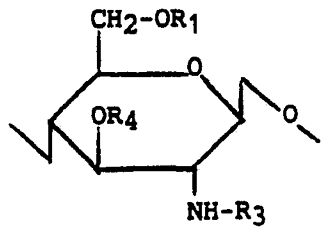

(Ia) (Ib)

dans lesquelles :

- $R_1$ représente un radical alkylcarbonyle contenant 2 à 4 atomes de carbone,
- $R_2$ représente un radical alkyle contenant 2 à 21 atomes de carbone ou un radical phényle éventuellement substitué par un ou plusieurs radicaux identiques ou différents choisis parmi les radicaux hydroxy ou alcoxy,
- $R_3$ représente un atome d'hydrogène ou un radical acétyle ou alkylcarbonyle contenant 2 à 4 atomes de carbone,
- $R_4$ représente un atome d'hydrogène ou un radical alkylcarbonyle contenant 2 à 4 atomes de carbone,

2 - Nouveaux dérivés selon la revendication 1 caractérisés en ce que leur masse moléculaire moyenne est comprise entre 10.000 et 110.000 et de préférence entre 10.000 et 25.000.

3 - Nouveaux dérivés selon la revendication 1 caractérisés en ce qu'ils sont composés de :
60 à 100 % de motifs répondant à la formule (Ia)
0 à 40 % de motifs répondant à la formule (Ib)

4 - Nouveaux dérivés selon la revendication 1 caractérisés en oe que $R_1$ et $R_3$ représentent le groupe acétyle.

5 - Nouveaux dérivés selon la revendication t caractérisés en ce que $R_2$ représente un groupe alkylé ayant 5 à 12 atomes de carbone.

6 - Nouveaux dérivés selon la revendication 1 caractérisés en ce que $R_4$ est identique à $R_1$.

7 - Procédé de préparation des nouveaux dérivés du chitosan selon la revendication 1 caractérisé en ce que :
- dans une première étape, on hydrolyse un chitosan désacétylé à plus de 80 %,
- dans une deuxième étape, on condense un aldéhyde aliphatique ou aromatique sur le chitosan issu de la première étape,
- dans une troisième étape, on estérifie le chitosan issu de la deuxième étape.

8 - Procédé selon la revendication 7 caractérisé en ce que l'hydrolyse est effectuée avec un acide fort choisi parmi l'acide chlorhydrique, l'acide sulfurique, l'acide nitrique, les acides minéraux supportés.

9 - Procédé selon la revendication 7 caractérisé en ce que l'on hydrolyse 25 à 50 g de chitosan par litre d'acide d'acidité 0,5 à 1 N.

10 - Procédé selon la revendication 7 caractérisé en ce que l'hydrolyse est effectuée à une température comprise entre 80 et 120° C.

11 - Procédé selon la revendication 7 caractérisé en ce que l'aldéhyde utilisé au cours de la deuxième étape répond à la formule $R_2$-CHO (II) dans laquelle $R_2$ représente un groupe alkyle ayant 3 à 14 atomes de carbone ou un groupe phényle substitué par un groupe hydroxy et/ou méthoxy.

12 - Procédé selon la revendication 7 à 11 caractérisé en ce que la deuxième étape est effectuée dans un solvant hydro-alcoolique.

13 - Procédé selon la revendication 7 et 11 à 12 caractérisé en ce que la condensation est effectuée à un pH compris entre 5,5 et 6.

14 - Procédé selon la revendication 7 caractérisé en ce que le rapport molaire de l'aldéhyde par rapport au monomère osidique moyen du chitosan hydrolysé est supérieur à 15 et de préférence d'environ 20.

15 - Procédé selon la revendication 7 caractérisé en ce que la condensation est effectuée à une température comprise entre et 50° C et de préférence entre 10 et 30° C.

16 - Procédé selon la revendication 7 caractérisé en ce que la troisième étape est réalisée avec un acide ou un dérivé d'acide de formule (III)

$(R\,CO)_nA$ (III)

dans laquelle :
n est égal à 1 ou 2

A est égal à un groupe hydroxyle ou un halogène quand n = 1
A est égal à l'oxygène quand n est égal à 2
R représente un groupe alkyle ayant 1 à 3 atomes de carbone et de préférence 1.

17 - Procédé selon la revendication 16 caractérisé en ce que le dérivé d'acide de formule (III) est l'anhydride acétique ou le chlorure d'acétyle.

18 - Procédé selon les revendications 7 et 16 caractérisé en ce que la condensation est effectuée dans la pyridine.

19 - Utilisation des chitosans selon la revendication 1 en cosmétologie.

20 - Utilisation des chitosans selon la revendication 1 pour la complexation des métaux.

21 - Utilisation des chitosans selon la revendication 1 pour la préparation de film, de fibres ou de revêtements.

22 - Compositions pour l'enrobage d'additifs alimentaires ou de substances biologiquement actives destinés à l'alimentation des ruminants qui sont stables à un pH supérieur à 5 et qui permettent la libération de la substance active à un pH inférieur à 3,5 caractérisées en ce qu'elles contiennent comme substance sensible aux variations de pH un dérivé selon l'une quelconque des revendications 1 à 6.

23 - Compositions selon la revendication 22 caractérisées en ce qu'elles sont constituées d'un dérivé organosoluble du chitosan selon l'une quelconque des revendications 1 à 6 en association avec une substance hydrophobe dont le point de fusion est supérieur à 60 °C.

24 - Granulés enrobés caractérisés en ce qu'ils sont constitués d'un noyau de substance active entouré d'une pellicule continue d'une composition d'enrobage contenant comme substance sensible aux variations du pH un dérivé organosoluble du chitosan tel que défini dans l'une des revendications 1 à 6.

25 - Granulés enrobés caractérisés en ce qu'ils sont constitués d'un noyau de substance active entouré d'une pellicule continue d'une composition d'enrobage selon la revendication 23.

26 - Granulés enrobés selon l'une des revendications 24 ou caractérisés en ce que la substance active est choisie parmi les médicaments, les vitamines et les acides aminés essentiels.

27 - Granulés enrobés selon l'une des revendications 24 ou 25 caractérisés en ce que la substance active est choisie parmi la méthionine ou la lysine.

Office européen des brevets

# RAPPORT DE RECHERCHE EUROPEENNE

Numero de la demande

EP 89 40 3307

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-4 424 346 (LAURANCE D. HALL) * Première page, résumé; colonne 14, revendication 1 * | 1,19,20 | C 08 B 37/08<br>A 23 K 1/16<br>A 23 K 1/18<br>A 61 K 7/00<br>C 02 F 1/28<br>D 01 F 9/00<br>D 06 M 15/00 |
| A | PATENT ABSTRACTS OF JAPAN, vol. 10, no. 172 (C-354)[2228], 18 juin 1986; & JP-A-61 21 102 (YAIZU SUISAN KAGAKU KOGYO K.K.) 29-01-1986 * Résumé * | 1,7-10 | |
| A | AGRICULTURAL AND BIOLOGICAL CHEMISTRY, vol. 47, no. 6, 1983, pages 1389-1391, Tokyo, JP; S. HIRANO et al.: "Some O-stearoyl derivatives of chitosan prepared via its Schiff's base intermediates" * En entier * | 1 | |
| A | CARBOHYDRATE RESEARCH, vol. 71, 1979, pages 344-348, Elsevier Scientific Publishing Co., Amsterdam, NL; S. HIRANO et al.: "N-methylenechitosan gels, and some of their properties as media for gel chromatography" * Page 344, tableau I * | 1 | **DOMAINES TECHNIQUES RECHERCHES (Int. Cl.5)**<br>C 08 B<br>A 23 K<br>A 61 K<br>C 02 F<br>D 01 F<br>D 06 M |
| D,A | FR-A-2 575 039 (AEC SOCIETE DE CHIMIE ORGANIQUE ET BIOLOGIQUE) * Revendications 1,6,12-15; exemples 18-13; page 5, lignes 32-34 * | 1,22-27 | |
| A | CHEMICAL ABSTRACTS, vol. 103, no. 25, 23 décembre 1985, page 772, résumé no. 213816c, Columbus, Ohio, US; & JP-A-60 130 346 (NIPPON SODA CO., LTD) 11-07-1985 * Résumé * | 1,22-27 | |

-/-

Le présent rapport a été établi pour toutes les revendications

| Lieu de la recherche | Date d'achèvement de la recherche | Examinateur |
|---|---|---|
| LA HAYE | 06-03-1990 | DEKEIREL M.J. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 89 40 3307

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | PATENT ABSTRACTS OF JAPAN, vol. 12, no. 181 (C-499)[3028], 27 mai 1988; & JP-A-62 288 602 (AGENCY OF IND. SCIENCE & TECHNOL.) 15-12-1987<br>* Résumé *<br>--- | 1 | |
| A | CARBOHYDRATE POLYMERS, vol. 3, no. 1, 1983, pages 53-75, Applied Science Publishers Ltd, Barking, Essex, GB; R.A.A. MUZZARELLI: "Chitin and its derivatives: New trends of applied research"<br>* Page 53, résumé *<br>----- | 1,20,21 | |

DOMAINES TECHNIQUES RECHERCHES (Int. Cl.5)

Le présent rapport a été établi pour toutes les revendications

| Lieu de la recherche | Date d'achèvement de la recherche | Examinateur |
|---|---|---|
| LA HAYE | 06-03-1990 | DEKEIREL M.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)